Europäisches Patentamt

European Patent Office

Office européen des brevets

(19)

(11) **EP 0 801 952 A2**

(12) **EUROPÄISCHE PATENTANMELDUNG**

(43) Veröffentlichungstag:
 **22.10.1997 Patentblatt 1997/43**

(51) Int. Cl.⁶: **A61L 2/14**

(21) Anmeldenummer: **97105600.7**

(22) Anmeldetag: **04.04.1997**

(84) Benannte Vertragsstaaten:
 **DE GB IT**

(30) Priorität: **20.04.1996 DE 19615735**

(71) Anmelder: **Rüdiger Haaga GmbH
 D-78727 Altoberndorf (DE)**

(72) Erfinder: **Kasper, Wolfgang, Dr.
 87452 Altusried (DE)**

(74) Vertreter: **Wilhelm & Dauster
 Patentanwälte
 European Patent Attorneys
 Hospitalstrasse 8
 70174 Stuttgart (DE)**

(54) **Vorrichtung zum Sterilisieren der Innenflächen von druck-empfindlichen Behältern**

(57)     Bei einer Vorrichtung zum Sterilisieren der Innenflächen von druckempfindlichen Behältern mittels ionisierter Teilchen ist eine evakuierbare Kammer vorgesehen, welche die Behälter aufnimmt. Die einem Behälter zugeordneten, über eine Wechselspannung angeregten Elektroden sind außerhalb des zugehörigen Behälters und diesen eng umschließend angeord- net. Dadurch wird zum einen erreicht, daß der Behälter von innen und außen dem gleichen Druck ausgesetzt ist, und zum anderen, daß die zu sterilisierende Innen- fläche des Behälters zugleich im wesentlichen die plas- mabegrenzende Wand ist.

Fig.1

EP 0 801 952 A2

## Beschreibung

Die Erfindung betrifft eine Vorrichtung zum Sterilisieren der Innenflächen von elektrisch nichtleitenden, druckempfindlichen, eine unverschlossene Füllöffnung aufweisenden Behältern mittels ionisierter Teilchen, mit einer evakuierbaren Kammer zur Aufnahme der Behälter, mit einer an die Kammer angeschlossenen Zuleitung für zu ionisierendes Gas, mit in der Kammer befindlichen, jeweils zwei einem Behälter zugeordneten Elektroden zum Erzeugen eines Plasmas sowie mit einem Wechselstromgenerator zum Anlegen einer Wechselspannung an die Elektroden.

Bei einer Vorrichtung dieser Art (DE 44 08 301 A1) durchlaufen die zu sterilisierenden Behälter nacheinander drei Unterdruckkammern, von denen die mittlere für eine Sterilisationsbehandlung der druckempfindlichen Behälter mittels eines Niedrigtemperatur-Plasmas vorgesehen ist. In jeden Behälter, der auf einer metallischen Palette steht, werden ein Elektrodenpol und eine Gasdüse eingeführt.

Die sterilisierende Wirkung eines Plasmas beruht sowohl auf einer mechanischen Zerstörung der Keime durch Ionenbeschuß als auch auf einer chemischen Zerstörung. Bei nur geringem Energiebedarf kann das Plasma in kleinste Oberflächenrisse und -löcher eindringen, wobei mit zunehmendem Unterdruck sich die Temperatur so weit verringern läßt, daß auch hitzeempfindliche Behälter behandelt werden können. Ein Nachteil der bekannten Vorrichtung liegt in der ungenügenden Homogenität des Plasmas, welches nicht nur die zu sterilisierenden Flächen, sondern auch die übrigen Flächen beaufschlagt. Infolgedessen ist bei der bekannten Vorrichtung eine Sterilisationszeit von zwei Minuten angegeben, damit ein ausreichender Sterilisationsquotient erreicht wird. Eine solche Sterilisationszeit ist für industrielle Anwendungen zu lang.

Es ist ferner bekannt (deutsche Offenlegungsschrift 21 06 736), die Innenflächen eines Behälters mit einem Niederdruckplasma auf der Basis von Chlor, Brom oder Jod zu sterilisieren. Bei einer Variante sind dabei die Behälter im Innern eines Reaktors angeordnet, der von einer Spule umgeben ist. Bei einer anderen Variante werden die Behälter im Innern eines Reaktors behandelt, wobei eine Elektrode im Behälter angeordnet ist und eine zweite Elektrode den Behälter umgibt. Bei einer dritten Variante ist der Behälter zugleich der Reaktor und von einer Spule direkt umgeben. Nachteilig ist, daß bei den ersten zwei Varianten die Behälter auch von außen mit dem Plasma beaufschlagt werden, so daß es im Innenraum der Behälter nicht besonders wirksam ist. Bei der letztgenannten Variante können nur druckunempfindliche Behälter sterilisiert werden, da das Vakuum lediglich im Innern des Behälters besteht.

Der Erfindung liegt die Aufgabe zugrunde, mit einer Vorrichtung der eingangs genannten Art die Innenflächen druckempfindlicher Behälter zu sterilisieren, wobei die zu sterilisierende Oberfläche im wesentlichen zugleich die plasmabegrenzende Wand ist.

Die Aufgabe wird dadurch gelöst, daß beide Elektroden außerhalb des zugehörigen Behälters und diesen eng umschließend angeordnet sind.

Durch die erfindungsgemäße Anordnung der Elektroden ist zum einen gewährleistet, daß innerhalb und außerhalb des Behälters der gleiche Unterdruck herrscht, und zum anderen, daß die zu sterilisierende Oberfläche zugleich im wesentlichen die plasmabegrenzende Wand ist, was zu einem guten Wirkungsgrad des Plasmas und zu einer kurzen Behandlungszeit führt. Bei dieser kapazitiven Anregung treffen die Ionen mit besonders hoher Energie auf die zu behandelnde Wand, was einen hohen Sterilisationsquotienten zur Folge hat, der wenigstens den Wert von $10^4$ erreicht. Dieser Sterilisationsquotient ist folgendermaßen definiert:

$$SQ = \log(a/b)$$

Mit a ist die Anzahl der Keime vor der Sterilisation, mit b die Anzahl der Keime nach der Sterilisation bezeichnet.

Bei dem erfindungsgemäßen engen Umschließen des Behälters durch die beiden Elektroden entsteht ein kleiner Spalt, der zwar den Unterdruck dort wirksam werden läßt, der jedoch aufgrund seiner kleinen Abmessungen verhindert, daß bereits außerhalb des Behälters, also dort, wo es nicht erwünscht ist, ein Plasma gezündet wird. Die Größe des Spaltes ist durch die sogenannte Debye-Länge bestimmt, die die charakteristische Abschirmlänge festlegt und bei praktischen Anwendungen der erfindungsgemäßen Art zwischen 100 und 500 µm beträgt. Dadurch wird an den tatsächlich zu sterilisierenden Flächen eine große Homogenität erreicht, so daß Sterilisationszeiten von wenigen Sekunden möglich werden. Der im Innern und außerhalb des Behälters wirksame Unterdruck macht es zum einen möglich, druckempfindliche Behälter zu sterilisieren, während andererseits aufgrund des hohen Unterdruckes die Behandlungstemperaturen so gering sind, daß auch hitzeempfindliche Behälter wie beispielsweise Papier behandelt werden können.

Als "druckempfindlich" werden im Zusammenhang mit der Erfindung nicht nur kompressible Behälter, beispielsweise aus Papier, sondern auch solche Behälter bezeichnet, die unter Druckeinwirkung nur sporadisch zerbrechen, beispielsweise Glasflaschen.

Zweckmäßig sind die Elektroden dem Behälter von zwei Seiten her zustellbar. Dies macht es nicht nur möglich, daß die Elektroden den Behälter erfindungsgemäß von außen umschließen, sondern der erforderliche kleine Abstand läßt sich auch auf einfache Weise dadurch einstellen, daß die Elektroden praktisch bis auf Anschlag an die Außenwandung des Behälters herangeführt werden. Das außerhalb des Behälters erforderliche Vakuum wird dadurch nicht beeinträchtigt.

Vorteilhaft kann wenigstens eine Elektrode mehreren Behältern zugeordnet sein. Dies führt zu einem verringerten apparativen Aufwand pro Behälter. Dabei kann beispielsweise eine längere Behälterreihe zwischen insgesamt lediglich zwei Elektroden angeordnet werden.

In Ausgestaltung der Erfindung haben die Elektroden in dem unmittelbar an die Füllöffnung angrenzenden Bereich eine Aussparung. Dadurch wird auch der unmittelbar an die Füllöffnung anschließende Außenbereich des Behälters mitsterilisiert. Dies ist insbesondere dann vorteilhaft, wenn die Aussparung zugleich auch für die Aufnahme eines der Füllöffnung zugeordneten Verschlußstückes vorgesehen ist. Bei diesem Verschlußstück kann es sich beispielsweise um ein sogenanntes Pull-tab handeln, welches bereits neben der Füllöffnung am Deckel des Behälters angebracht ist. Es erübrigt sich dann, Behälter und Verschlußstücke gesondert zu sterilisieren.

Ein ganz besonderer Vorteil der Erfindung liegt darin, daß das evakuierbare Volumen der Kammer im wesentlichen nur aus dem Inneren der Behälter besteht. Durch die geschickte Anordnung der Elektroden und die diese umgebende Wand der Kammer läßt sich der zu evakuierende Raum der Kammer, soweit er sich außerhalb der Behälter befindet, äußerst klein halten. Obwohl also die Behälter von einer evakuierbaren Kammer umgeben sind, ist die praktische Auswirkung ähnlich, als wenn die Behälter selbst den Reaktor bilden würden. Man braucht daher nur sehr wenig Luft abzupumpen.

Zweckmäßig soll die Kammer unter einem Betriebsdruck von 1 bis 100 Pa stehen. Dieser niedrige Druck hat eine niedrige Gastemperatur zur Folge, wie sie beispielsweise für Papierdosen benötigt wird. Der niedrige Betriebsdruck hat des weiteren zur Folge, daß die Behandlungszeit bis auf wenige Sekunden verringert wird.

Als Behandlungsgase wählt man zweckmäßig ungiftige Gase, wobei bevorzugt Sauerstoff, Wasserstoff, Stickstoff, Helium, Neon, Argon, Krypton, Wasserdampf, Wasserstoffperoxiddampf oder Gemische der Gase in Betracht kommmen. Es hat sich jedoch als besonders vorteilhaft erwiesen, als ionisierbares Gas Wasserstoff oder Helium zu verwenden. Diese Gase bilden im Plasma die leichtesten und kleinstmöglichen Ionen, die selbst in kleinste Poren des Behälters eindringen können und am besten zur Oberfläche diffundieren, und das bei hoher Ionisationsenergie.

Zweckmäßig wird für die Wechselspannung eine Hochfrequenz vorgesehen, die zwischen den Plasmafrequenzen der Ionen und Elektronen liegt. Es läßt sich daher die zugelassene Hochfrequenz von 13,56 Mhz verwenden.

In besonders vorteilhafter Ausgestaltung der Erfindung ist die Kammer insgesamt als sogenannter Parallelplatten-Reaktor ausgebildet. Dadurch wird es möglich, eine größere Anzahl von Behältern zu gleicher Zeit auf besonders wirtschaftliche Art zu sterilisieren. Werden beispielsweise zwanzig Behälter gleichzeitig bei einer Sterilisationszeit von 16 Sekunden sterilisiert, dann läßt sich auf diese Art die Taktzeit pro Behälter auf unter eine Sekunde verringern.

Weitere Vorteile und Merkmale der Erfindung ergeben sich aus der nachfolgenden Beschreibung eines schematisch dargestellten Ausführungsbeispieles. Es zeigen:

Figur 1 eine geschnittene Seitenansicht auf eine als Parallelplatten-Reaktor ausgebildetete Vorrichtung zum Sterilisieren von druckempfindlichen Behältern,

Figur 2 einen Schnitt durch die Vorrichtung längs der Schnittfläche II-II der Figur 1,

Figur 3 in vergrößerter Darstellung eine Detailansicht der Figur 1 im Bereich einer Füllöffnung des zu sterilisierenden Behälters.

In den Figuren ist eine evakuierbare Kammer 1 nur strichpunktiert als Kontur angedeutet. Sie soll, wie dies ja bei sehr unterschiedlichen Reaktoren bekannt ist, so ausgebildet sein, daß die zu sterilisierenden Behälter 3 nach dem öffnen der Kammer 1 in dieselbe eingebracht werden können, wobei im Anschluß danach die Kammer 1 abgedichtet verschließbar sein muß. Zweckmäßig ist dabei die Kammer 1 einer nicht dargestellten Transporteinrichtung zugeordnet, die beispielsweise die Behälter 3 von oben zuführt und in die Kammer 1 einbringt und nach unten wieder aus der Kammer 1 entfernt. Für diese Handhabung muß sich die evakuierbare Kammer 1, welche bei Betrieb nach außen abgedichtet sein muß, auf einfache Weise öffnen und schließen lassen. Derartige Reaktoren sind grundsätzlich durch den Stand der Technik bekannt. Die Kammer 1 ist durch eine Vakuumpumpe 2 bis auf einen Unterdruck von 1 bis ca. 100 Pa evakuierbar.

Die Kammer 1 dient der gleichzeitigen Aufnahme von einer Vielzahl, beispielsweise 20, Behältern 3, deren Innenflächen 4 sterilisiert werden sollen. Insbesondere handelt es sich um Behälter 3, die elektrisch nichtleitend und zudem druckempfindlich sind, beispielsweise Behälter 3 aus Papier. Jeder dieser Behälter 3 weist eine Füllöffnung 5 auf, die während des Sterilisierens nicht verschlossen ist. Über diese Füllöffnung 5 kann nach dem Sterilisieren ein Füllgut eingefüllt werden, wonach die Füllöffnung 5 verschlossen wird.

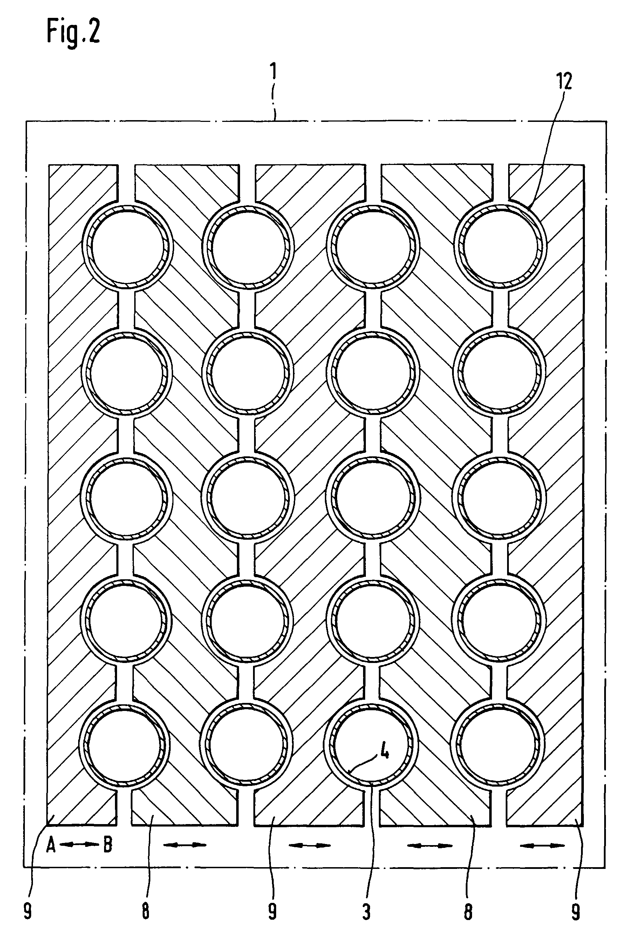

Wie insbesondere aus Figur 2 ersichtlich ist, sind die in der Kammer 1 zu sterilisierenden Behälter 3 in mehreren Reihen nebeneinander angeordnet.

An die Kammer 1 ist eine Zuleitung 6 für zu ionisierendes Gas, beispielsweise Wasserstoff, angeschlossen. Der Zustrom dieses Gases läßt sich über ein Drosselventil 7 regulieren.

Im Innern der evakuierbaren Kammer 1 gibt es mehrere, zu den Reihen der Behälter 3 parallel verlaufende Elektroden 8 und 9, von denen die Elektroden 8 an eine Wechselspannung angeschlossen und die Elektroden 9 geerdet sind. Obwohl jede dieser Elektroden 8, 9 einer Vielzahl von Behältern 3 zugeordnet ist, gehören andererseits zu jedem Behälter 3 zwei Elektroden 8 und 9. Die Kammer 1 ist in nicht dargestellter Weise so ausgebildet, daß sich die Elektroden 8 und 9 entsprechend den Richtungen der Pfeile A und B relativ zueinander verstellen lassen, so daß die Behälter 3 unbehindert in die Kammer 1 einführbar sind, wonach die Elektroden 8 und 9 von zwei Seiten her den Behältern 3 zustellbar sind.

Die Kontur der Elektroden 8 und 9 ist der Außenkontur der Behälter 3 derart angepaßt, daß die Behälter 3 von den Elektroden 8 und 9 nahezu spaltfrei eng umschlossen werden können. Beispielsweise können die Behälter 3 zylindrisch ausgestaltet sein. Es kann sich jedoch in nicht dargestellter Weise auch um andere Konturen handeln, wie sie beispielsweise bei Flaschen üblich sind.

Die Elektroden 8 sind an einen hochfrequenten Wechselstromgenerator 10 angeschlossen, und zwar unter Zwischenschaltung einer sogenannten Matchbox 11, welche die Wechselspannung vom Wechselstromgenerator 10 in das zu bildende Plasma überträgt. Die Matchbox 11 dient dem Zweck, die Impedanz des Plasmas an den Wechselstromgenerator 10 anzupassen.

Nach dem Zustellen der Elektroden 8 und 9 seitlich an die Behälter 3 entsteht jeweils ein enger Spalt 12, der es möglich macht, daß sich das durch die Vakuumpumpe 2 erzeugte Vakuum nicht nur im Innern der Behälter 3, sondern auch um die Behälter 3 herum ausbildet. Dies ist für druckempfindliche Behälter 3 zwingend erforderlich. Der Spalt 12 soll jedoch so eng sein, daß er kleiner als die bereits erläuterte Debye-Länge ist, so daß sich außerhalb der Behälter 3, d. h. zwischen den Elektroden 8, 9 und der Außenwandung der Behälter 3, noch kein Plasma bilden kann. Vielmehr soll das Plasma praktisch ausschließlich im Innern der Behälter 3 gezündet werden, also dort, wo sich die zu sterilisierenden Innenflächen 4 befinden. Durch die Anordnung der Elektroden 8 und 9, die zum einen außerhalb der Behälter 3 angeordnet sind und zum anderen die Behälter 3 eng umschließen, wird es somit einerseits möglich, daß innerhalb und außerhalb der Behälter 3 der gleiche Unterdruck herrscht, und andererseits möglich, daß die Innenflächen 4 der Behälter 3 praktisch ausschließlich die plasmabegrenzende Wand bilden. Dies führt in Verbindung mit dem gewählten Unterdruck zu ausreichend kurzen Sterilisationszeiten.

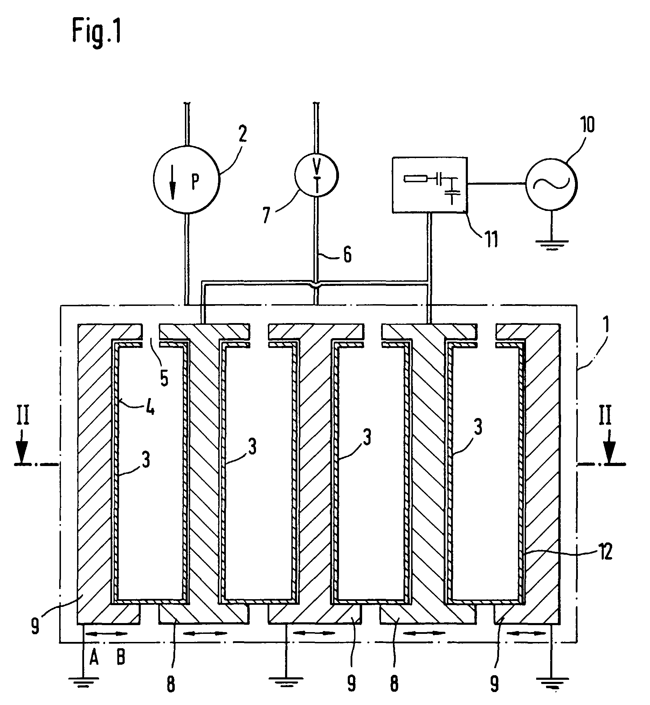

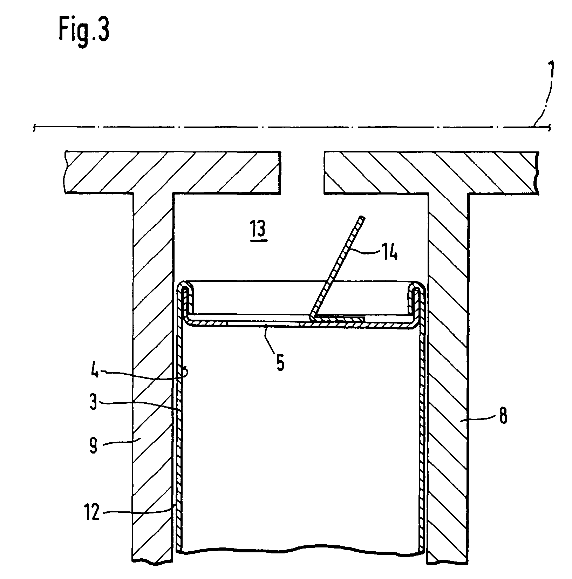

Abweichend von der Ausführung nach Figur 1 kann gemäß der vergrößerten Darstellung nach Figur 3 vorgesehen sein, daß die Elektroden 8 und 9 im Bereich der Füllöffnung 5 eine kleine Aussparung 13 bilden, was zur Folge hat, daß im Bereich der Füllöffnung 5 auch außerhalb des Behälters 3 ein Plasma gezündet werden kann. Dies ist insbesondere dann vorteilhaft, wenn der Füllöffnung 5 ein Verschlußstück 14, beispielsweise ein sogenanntes Pull-tab, zugeordnet ist, das mitsterilisiert werden soll.

Nach der Plasma-Sterilisation, wenn das Innere der Kammer 1 wieder unter normalem Druck steht, kann in nicht dargestellter Weise es sogar möglich gemacht werden, die Behälter 3 noch innerhalb der Kammer 1 mit einem Füllgut zu versehen. Zu diesem Zwecke müßte in nicht dargestellter Weise noch ein Füllrohr in die Kammer 1 eingeführt werden. Dies würde dann dazu führen, daß die Behälter 3 im Innern der Kammer 1 sterilisiert, abgefüllt und verschlossen werden können.

Die Elektroden 8 und 9 in Verbindung mit der Wandung der Kammer 1 lassen sich so anordnen, daß das evakuierbare Volumen der Kammer 1 im wesentlichen nur aus dem Inneren der Behälter 3 besteht, so daß zum Sterilisieren nur sehr wenig Luft abgepumpt zu werden braucht. Während des Sterilisierens steht die Kammer 1 unter einem Betriebsdruck, der sich zwischen den Werten 1 und 100 Pa einstellen läßt. Dies hat gleichzeitig eine ausreichend niedrige Gastemperatur zur Folge, die nur geringfügig über der Raumtemperatur liegt.

Damit vorzugsweise leichte Ionen mit hoher Ionisationsenergie erzeugt werden, wird als Gas vorrangig Wasserstoff oder Helium verwendet. Für die an die Elektroden 8 und 9 anzulegende Wechselspannung ist eine Hochfrequenz von beispielsweise 13,56 Mhz vorgesehen.

Wie insbesondere aus Figur 2 ersichtlich, ist die evakuierbare Kammer 1 als Parallelplatten-Reaktor ausgebildet, wobei die Elektroden 8 und 9 entsprechend den Pfeilrichtungen A und B den Behältern 3 zustellbar sind. Die Länge der Elektroden 8 und 9 entspricht dabei der Länge einer Reihe von Behältern 3.

**Patentansprüche**

1. Vorrichtung zum Sterilisieren der Innenflächen von elektrisch nichtleitenden, druckempfindlichen, eine unverschlossene Füllöffnung aufweisenden Behältern mittels ionisierter Teilchen, mit einer evakuierbaren Kammer zur Aufnahme der Behälter, mit einer an die Kammer angeschlossenen Zuleitung für zu ionisierendes Gas, mit in der Kammer befindlichen, jeweils zwei einem Behälter zugeordneten Elektroden zum Erzeugen eines Plasmas sowie mit einem Wechselstromgenerator zum Anlegen einer Wechselspannung an die Elektroden, dadurch gekennzeichnet, daß beide Elektroden (8,9) außerhalb des zugehörigen Behälters (3) und diesen eng umschließend angeordnet sind.

2. Vorrichtung nach Anspruch 1, dadurch gekennzeichnet, daß die Elektroden (8,9) dem Behälter (3) von zwei Seiten her zustellbar sind.

3. Vorrichtung nach Anspruch 1 oder 2, dadurch gekennzeichnet, daß wenigstens eine Elektrode (8,9) mehreren Behältern (3) zugeordnet ist.

4. Vorrichtung nach einem der Ansprüche 1 bis 3, dadurch gekennzeichnet, daß die Elektroden (8,9) in dem unmittelbar an die Füllöffnung (5) angrenzenden Bereich eine Aussparung (13) haben.

5. Vorrichtung nach Anspruch 4, dadurch gekennzeichnet, daß die Aussparung (13) für die Aufnahme eines der Füllöffnung (5) zugeordneten Verschlußstückes (14) vorgesehen ist.

6. Vorrichtung nach einem der Ansprüche 1 bis 5, dadurch gekennzeichnet, daß das evakuierbare Volumen der Kammer (1) im wesentlichen aus dem Inneren der Behälter (3) besteht.

7. Vorrichtung nach einem der Ansprüche 1 bis 6, dadurch gekennzeichnet, daß die Kammer (1) unter einem Betriebsdruck von 1 bis 100 Pa steht.

8. Vorrichtung nach einem der Ansprüche 1 bis 7, dadurch gekennzeichnet, daß als ionisierbares Gas Wasserstoff oder Helium vorgesehen ist.

9. Vorrichtung nach einem der Ansprüche 1 bis 8, dadurch gekennzeichnet, daß für die Wechselspannung eine Hochfrequenz von 13,56 Mhz vorgesehen ist.

10. Vorrichtung nach einem der Ansprüche 1 bis 9, dadurch gekennzeichnet, daß die Kammer (1) als sogenannter Parallelplatten-Reaktor ausgebildet ist.

# Fig.1

# Fig.2

# Fig.3